# EUROPEAN PATENT APPLICATION

(11) **EP 3 495 041 A1**
(43) Date of publication of application: **12.06.2019**
(21) Application number: 17836662.1
(22) Date of filing: 04.07.2017
(51) Int. Cl.: B01J 23/889, B01J 37/04, B01J 37/08, C07C 1/04, C07C 1/12, C07C 9/04, C10L 3/08, C07B 61/00

(54) **METHANATION REACTION CATALYST, METHOD FOR PRODUCING METHANATION REACTION CATALYST, AND METHOD FOR PRODUCING METHANE**

(30) Priority: 02.08.2016 JP 2016152107
(71) Applicant: Hitachi Zosen Corporation, Osaka-shi, Osaka 559-8559 (JP)
(72) Inventor: TAKANO, Hiroyuki, Osaka-shi Osaka 559-8559 (JP); KIRIHATA, Yuki, Osaka-shi Osaka 559-8559 (JP); IZUMIYA, Kouichi, Osaka-shi Osaka 559-8559 (JP); NISHIDA, Yusuke, Osaka-shi Osaka 559-8559 (JP)
(74) Representative: Grünecker Patent- und Rechtsanwälte PartG mbB
(86) International application number: PCT/JP2017/024538
(87) International publication number: WO 2018/025555

(57) **Abstract**

The methanation reaction catalyst is a methanation reaction catalyst for methanation by allowing CO and/or CO₂ to react with hydrogen, wherein the methanation reaction catalyst includes a stabilized zirconia support, into which a stabilizing element forms a solid solution, and having a crystal structure of a tetragonal system and/or a cubic system, and Ni supported on the stabilized zirconia support. The stabilizing element is a transition element of at least one selected from the group consisting of Mn, Fe, and Co.

## Description

### [TECHNICAL FIELD]

The present invention relates to a methanation reaction catalyst, a method for producing a methanation reaction catalyst, and a method for producing methane.

### [BACKGROUND ART]

A methanation reaction catalyst for methanation by allowing CO or CO₂ to react with hydrogen has been known. Patent Document 1 has proposed, for such a methanation reaction catalyst, for example, a catalyst represented by MnₐZr_{b}Ni_{c}Oₓ, a being 0.1 to 5 mol%, b being 3 to 20 mol%, and c being 100 - (a + b)mol% and containing a metal-doped nickel oxide (see Patent Document 1 below).

Such a catalyst is prepared by dissolving salts of the starting materials in an alcohol solvent containing a complexing agent, and then the mixture is aged to prepare a gel, and then the gel is dried, and calcined at 200 to 400°C.

### [Citation List]

### [Patent Document]

Patent Document 1: Japanese Translation of PCT International Application Publication No. 2010-520807

### [SUMMARY OF THE INVENTION]

### [PROBLEM TO BE SOLVED BY THE INVENTION]

Effective use of the methane produced by methanation reaction as an energy source has been examined. However, the catalyst described in Patent Document 1 is for removing carbon monoxide from hydrogen-containing gas used for fuel cells, and cannot efficiently produce methane, and improvement in methane yield per unit mass is limited.

An object of the present invention is to provide a methanation reaction catalyst with which a methane yield per unit mass can be improved, a method for producing a methanation reaction catalyst, and a method for producing methane.

### [MEANS FOR SOLVING THE PROBLEM]

The present invention [1] includes a methanation reaction catalyst for methanation by allowing CO and/or CO₂ to react with hydrogen, wherein the methanation reaction catalyst includes a stabilized zirconia support, into which a stabilizing element forms a solid solution, and having a crystal structure of a tetragonal system and/or a cubic system, and Ni supported on the stabilized zirconia support, and the stabilizing element is a transition element of at least one selected from the group consisting of Mn, Fe, and Co.

The catalyst described in Patent Document 1 is prepared by calcining a gel at 400°C or less, and is amorphous.

Meanwhile, with the above-described configuration, a specific transition element forms a solid solution into the stabilized zirconia support, and the stabilized zirconia support has a crystal structure of a tetragonal system and/or a cubic system. Such a methanation reaction catalyst can improve the methane yield per unit mass compared with the case where the stabilized zirconia support is amorphous.

The present invention [2] includes the methanation reaction catalyst described in [1] above, wherein relative to a total of Zr composing the stabilized zirconia support, the stabilizing element, and Ni, the atom percentage of Zr is 6.5 atom% or more and 66.5 atom% or less, the atom percentage of the stabilizing element is 0.50 atom% or more and 24.5 atom% or less, and the atom percentage of Ni is 30.0 atom% or more and 90.0 atom% or less.

With this configuration, the atom percentage of Zr, stabilizing element, and Ni is in the above-described range, and therefore the methane yield per unit mass can be reliably improved.

The present invention [3] includes the methanation reaction catalyst described in [1] or [2] above, wherein the Ni is supported on the stabilized zirconia support, and also forms a solid solution into the stabilized zirconia support.

With this configuration, Ni is supported on the stabilized zirconia support, and also forms a solid solution into the stabilized zirconia support. When Ni forms a solid solution into the stabilized zirconia support, because Zr ion (Zr⁴⁺) has a valency of 4, and Ni ion (Ni²⁺) has a valency of 2, oxygen defect (deficiency) is caused, and oxygen vacancy is formed in the crystal structure of the stabilized zirconia support.

The oxygen vacancy attracts oxygen atom of CO and/or CO₂ in the methanation reaction, and therefore on the surface of the methanation reaction catalyst, CO and/or CO₂ can be allowed to react with hydrogen efficiently.

The present invention [4] includes the methanation reaction catalyst described in any one of the above-described [1] to [3], wherein the stabilizing element is Mn.

With this configuration, the stabilizing element is Mn, and therefore improvement in the methane yield per unit mass can be achieved more reliably compared with the case where the stabilizing element is Fe and Co.

The present invention [5] includes the methanation reaction catalyst described in [4] above, wherein Mn includes at least Mn³⁺.

With this configuration, Mn forming a solid solution in the stabilized zirconia support contains at least Mn³⁺, and therefore oxygen vacancy can be reliably formed in the stabilized zirconia support.

The present invention [6] includes the methanation reaction catalyst described in any one of the above-described [1] to [5], wherein the atomic ratio of the stabilizing element/(Zr + the stabilizing element) is 0.05 or more and 0.35 or less.

With this configuration, the atomic ratio of the stabilizing element/(Zr + the stabilizing element) is in the above-described range, and therefore the methane yield per unit mass can be improved even more reliably.

The present invention [7] includes a method for producing methane, including allowing the methanation reaction catalyst described in any one of the above-described [1] to [6] to contact a gas mixture containing CO and/or CO₂ and hydrogen gas at 200°C or more.

With this method, the above-described methanation reaction catalyst is allowed to contact with a gas mixture containing CO and/or CO₂, and hydrogen gas at 200°C or more, and therefore CO and/or CO₂ can be allowed to react with hydrogen gas efficiently, and methane can be produced efficiently.

The present invention [8] includes a method for producing a methanation reaction catalyst, the method including the steps of: preparing a mixture by mixing zirconia and/or Zr salt, a salt of the stabilizing element, and Ni salt, and calcining the mixture at 550°C or more and 800°C or less, wherein the stabilizing element is a transition element of at least one selected from the group consisting of Mn, Fe, and Co.

With this method, the methanation reaction catalyst can be produced by mixing zirconia and/or Zr salt, a specific transition element salt, and Ni salt, and then calcining the mixture at 550°C or more and 800°C or less. The methanation reaction catalyst which allows for improvement in the methane yield per unit mass can be produced with an easy method.

### [Effects of the Invention]

With the methanation reaction catalyst of the present invention, the methane yield per unit mass can be improved.

With the method for producing methane of the present invention, CO and/or CO₂ can be allowed to react with hydrogen gas efficiently, and methane can be produced efficiently.

With the method for producing a methanation reaction catalyst of the present invention, a methanation reaction catalyst which allows for improvement in the methane yield per unit mass can be produced with an easy method.

### [BRIEF DESCRIPTION OF THE DRAWINGS]

FIG. 1 is a graph illustrating correlation between reaction temperature of the methanation reaction of CO₂ and the methane yield per unit mass in the presence of the methanation reaction catalyst of Examples 1 to 3 and Comparative Example 1.
FIG. 2 is an X-ray diffraction pattern of the methanation reaction catalyst of Example 2 and Comparative Example 1.
FIG. 3 is a graph illustrating correlation between the calcination temperature and the BET specific surface area of the methanation reaction catalyst of Examples 1 to 3.
FIG. 4 is a graph illustrating correlation between the M/(Zr + M) atomic ratio and the methane yield per unit mass of the methanation reaction catalyst of Examples 2, 4 to 9, 17 to 20 and Comparative Examples 2 to 8.
FIG. 5 is a graph illustrating correlation between the M/(Zr + M) atomic ratio and the methane yield per unit mass of the methanation reaction catalyst of Examples 10 to 16 and Comparative Examples 9 to 14.
FIG. 6 is an X-ray diffraction pattern of the methanation reaction catalyst of Examples 2, and 4 to 9.
FIG. 7 is an X-ray diffraction pattern of the methanation reaction catalyst of Examples 17 to 20.
FIG. 8 is a graph illustrating correlation between the Mn/(Zr + Mn) atomic ratio and the crystal lattice spacing of the [111] planes of the stabilized zirconia support of the methanation reaction catalyst of Examples 2, 4 to 9, 17 to 20 and Comparative Examples 15 to 21.
FIG. 9 is a graph illustrating correlation between the Ni atom% and the methane yield per unit mass of the methanation reaction catalyst of Examples 2, 12, 21 to 24 and Comparative Examples 6, 12, 22 to 25.
FIG. 10 is a graph illustrating correlation between the M/(Zr + M) atomic ratio and methane yield per unit mass of the methanation reaction catalyst of Examples 2, 4 to 9, 25 to 32 and Comparative Examples 2 to 8.
FIG. 11 is a graph illustrating correlation between the reaction temperature of the methanation reaction of CO and the methane yield per unit mass in the presence of the methanation reaction catalyst of Examples 2, 26, 30 and Comparative Examples 6 and 26.
FIG. 12 is an X-ray diffraction pattern of the methanation reaction catalyst of Examples 25 to 28.
FIG. 13 is an X-ray diffraction pattern of the methanation reaction catalyst of Examples 29 to 32.
FIG. 14 is a graph illustrating correlation between the M/(Zr + M) atomic ratio and the crystal lattice spacing of the [111] planes of the stabilized zirconia support of the methanation reaction catalyst of Examples 2, 4 to 9, and 25 to 32.
FIG. 15 is an XPS spectrum (Mn 2p peak) of the methanation reaction catalyst of Comparative Examples 16 to 19.
FIG. 16 is an XPS spectrum (Mn 3s peak) of the methanation reaction catalyst of Comparative Example 18.

### [Description of the embodiments]

### 1. First embodiment

### (1-1) Methanation reaction catalyst

The methanation reaction catalyst is a methanation reaction catalyst for allowing CO and/or CO₂ to react with hydrogen for methanation, and includes a stabilized zirconia support and Ni supported on the stabilized zirconia support.

In the stabilized zirconia support, at least the stabilizing element and Zirconia form a solid solution, and the stabilized zirconia support has a crystal structure (unit lattice) of a tetragonal system and/or cubic system mainly composed of Zr. The crystal structure of the stabilized zirconia support is composed with Zr as a main component (fundamental component), and mainly Zr ions (Zr⁴⁺) are disposed at a plurality of lattice points of the crystal structure of the stabilized zirconia support.

The stabilizing element stabilizes the crystal structure of the stabilized zirconia support to be the tetragonal system and/or cubic system. The stabilizing element is a transition element having a smaller ionic radius than that of Zr ions (Zr⁴⁺). To be specific, the stabilizing element is a transition element of at least one selected from the group consisting of Mn, Fe, and Co.

In the stabilized zirconia support of the first embodiment, in addition to the stabilizing element and Zirconia, Ni forms a solid solution.

When the stabilizing element and Ni form solid solutions into the stabilized zirconia support, a portion of lattice points of the plurality of lattice points of the crystal structure are replaced, from Zr ions, with any of the above-described transition element ions (Mn ion, Fe ion, and Co ion) and Ni ion.

That is, the stabilizing element forming a solid solution into the stabilized zirconia support means, Zr ions disposed at the lattice points in the crystal structure are replaced with the above-described transition element ions, and Ni forming a solid solution into the stabilized zirconia support means, Zr ions disposed at the lattice points in the crystal structure are replaced with Ni ions.

Such a stabilized zirconia support contains Zr, the above-described transition element, Ni, and O, and preferably consists of Zr, the above-described transition element, Ni, and O. To be more specific, the stabilized zirconia support is represented by the general formula (1) below.

### General formula (1):

[Chemical Formula 1]

Zr⁴⁺_{1-(x+y)}M^{α+}ₓNi²⁺_{y}O_{2-((2-α/2)x+y)} (1)

(in formula (1), x and y are less than 1, and x + y is less than 1. M is one transition element selected from the group consisting of Mn, Fe, and Co, α is a valence of transition element ion of an integer of 2 or more and 4 or less)

In general formula (1), x represents, for example, 0.133 or more, and less than 1, preferably 0.248 or less. In general formula (1), y represents, for example, 0.010 or more, and less than 1, preferably 0.050 or less.

In general formula (1), M is the above-described transition element, and α represents valence of the transition element ion. To be more specific, when the transition element is Mn, for M^{α+}, Mn³⁺ and Mn⁴⁺ are used, preferably, Mn³⁺ is used. When the transition element is Fe, for M^{α+}, Fe²⁺ and Fe³⁺ are used, and preferably, Fe³⁺ is used. When the transition element is Co, for M^{α+}, Co²⁺ and Co³⁺ are used, and preferably, Co²⁺ is used.

At the plurality of lattice points of the stabilized zirconia support represented by general formula (1), any one of Zr ion, the above-described transition element ion, and Ni ion is disposed. The crystal structure of such a stabilized zirconia support preferably includes a perovskite structure.

The stabilized zirconia support has oxygen vacancies. The oxygen vacancies are formed by the following: the transition element and/or Ni form a solid solution into the stabilized zirconia support, and the transition element ion with a valence of 3 or less (divalent or trivalent) and/or Ni ion is replaced with Zr ion.

To be specific, when a trivalent transition element ion is replaced with Zr ion, based on Kröger-Vink formula represented by formula (2) below, oxygen vacancy is formed in the stabilized zirconia support, and when a divalent transition element ion or Ni ion is replaced with Zr ion, based on Kröger-Vink formula represented by formula (3) below, oxygen vacancy is formed in the stabilized zirconia support. 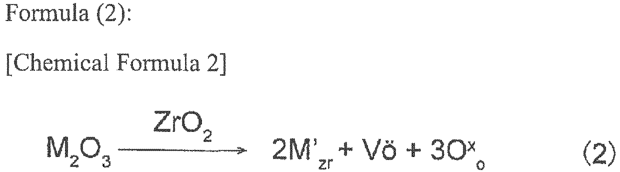 (in formula (2), M represents the transition element of M in general formula (1), and Vö represents oxygen vacancy) 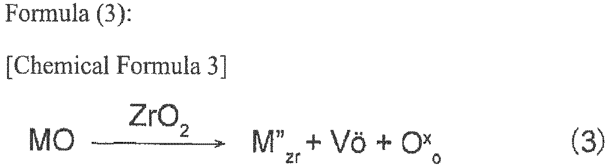 (in formula (3), M represents transition element of M in general formula (1) or Ni, and Vö represents oxygen vacancy)

When the tetravalent transition element ion is replaced with Zr ion, because their valences are the same, no oxygen vacancy is formed in the stabilized zirconia support.

The stabilized zirconia support can be used singly, or can be used in combination of two or more.

Of these examples of the stabilized zirconia support, in view of catalytic activity, preferably, the stabilized zirconia support with transition element of Mn (to be specific, stabilized zirconia support represented by general formula (4) and (5)) is used, even more preferably, the stabilized zirconia support represented by general formula (4) and (5) are used in combination. In general formula (4) and (5) below, the oxygen vacancy to be formed is also shown.

### General formula (4):

[Chemical Formula 4]

Zr⁴⁺_{1-(x+y)}Mn³⁺ₓNi²⁺_{y}O_{2-(0.5x+y)}Vö_{(0.5x+y)} (4)

(in formula (4), x and y are the same range as that of x and y of general formula (1), and Vö represents oxygen vacancy)

### General formula (5):

[Chemical Formula 5]

Zr⁴⁺_{1-(x+y)}Mn⁴⁺ₓNi²⁺_{y}O_{2-y}Vö_{y} (5)

(in formula (5), x and y are same range as x and y of general formula (1), and Vö represents oxygen vacancy)

The crystal lattice spacing of the stabilized zirconia support changes depending on the amounts of the stabilizing element and Ni forming the solid solution into the stabilized zirconia support, because the ionic radius of the stabilizing element ions and Ni ions is smaller than the ionic radius of Zr⁴⁺. The ionic radius of Zr⁴⁺, Ni²⁺, Mn³⁺, and Mn⁴⁺ is shown below for reference.
Zr⁴⁺:0.079nm
Ni²⁺:0.069nm
Mn³⁺:0.072nm
Mn⁴⁺:0.067nm

To be more specific, when the stabilizing element ions and Ni ions having smaller ionic radius than that of Zr ions are incorporated more in the crystal structure (that is, when x and y increase in general formula (1)), the crystal lattice spacing in the stabilized zirconia support decreases.

The lattice spacing of the [111] planes of the crystal structure of the stabilized zirconia support is, for example, 0.2920 nm or more, preferably 0.2930 nm or more, and for example, 0.2960 nm or less, preferably 0.2955 nm or less. The lattice spacing of the [111] planes of the crystal structure of the stabilized zirconia where the stabilizing element and Ni are not forming the solid solution is, in the case of tetragonal zirconia, 0.2975 nm, and in the case of cubic zirconia, 0.2965 nm.

In the case of methanation reaction catalyst, Ni is supported on the above-described stabilized zirconia support.

Ni can be NiO, or a metal state Ni, but in view of catalytic activity, preferably, a metal state Ni is used.

The methanation reaction catalyst in the first embodiment contains Zr composing the stabilized zirconia support, the stabilizing element forming the solid solution into the stabilized zirconia support, Ni forming a solid solution into the stabilized zirconia support, and Ni supported on the stabilized zirconia support.

That is, Ni is supported on the stabilized zirconia support, and also forms the solid solution into the stabilized zirconia support. In the following, Ni means a total of the Ni supported on the stabilized zirconia support and the Ni forming the solid solution in the stabilized zirconia support.

In the methanation reaction catalyst, relative to a total of Zr, the stabilizing element, and Ni (hereinafter referred to as a total of all the atoms), the atom percentage of Zr (=Zr/(Zr + stabilizing element + Ni) × 100) is, for example, 6.5 atom% or more, preferably 20 atom% or more, and for example, 66.5 atom% or less, preferably 50 atom% or less. The atom percentage of atoms in the methanation reaction catalyst is calculated based on the material component (zirconia and/or Zr salt, salt of the stabilizing element, and Ni salt) used in the method for producing a methanation reaction catalyst described later.

When the atom percentage of Zr is within the above-described range, the crystal structure of the tetragonal system and/or cubic system can be reliably formed in the stabilized zirconia support.

In the methanation reaction catalyst, relative to a total of all the atoms, the atom percentage of the stabilizing element (=stabilizing element/(Zr + stabilizing element + Ni) × 100) is, for example, 0.5 atom% or more, preferably 1.0 atom% or more, more preferably 2.0 atom% or more, and for example, 24.5 atom% or less, preferably 20 atom% or less, more preferably 7.0 atom% or less.

When the atom percentage of the stabilizing element is within the above-described range, the crystal structure of the tetragonal system and/or cubic system can be stabilized reliably.

In the methanation reaction catalyst, atom percentage of Ni relative to a total of all the atoms (=Ni/(Zr + stabilizing element + Ni) × 100) is, for example, 30.0 atom% or more, preferably 50.0 atom% or more, and for example, 90.0 atom% or less, preferably 70.0 atom% or less.

When the atom percentage of Ni is the above-described lower limit or more, improvement in catalytic activity can be reliably achieved, and when the atom percentage of Ni is the above-described upper limit or less, Ni coagulation and decline in Ni dispersiveness can be suppressed.

In the methanation reaction catalyst, the atomic ratio of stabilizing element/(Zr + stabilizing element) is, for example, 0.05 or more, and for example, 0.50 or less, preferably 0.35 or less, more preferably 0.20 or less, particularly preferably 0.15 or less.

When the atomic ratio of the stabilizing element/(Zr + stabilizing element) is within the above-described range, improvement in catalytic activity can be reliably achieved, and improvement in the methane yield per unit mass can be reliably achieved.

The form of the methanation reaction catalyst is not particularly limited, preferably, the methanation reaction catalyst is in the form of particles. When the methanation reaction catalyst is in the form of particles, the methanation reaction catalyst has an average secondary particle size of, for example, 7 µm or more, preferably 10 µm or more, and for example, 100 µm or less, preferably 70 µm or less. The average secondary particle size can be measured in accordance with the electron microscopy (JIS H7803:2005).

The methanation reaction catalyst has a specific surface area (BET specific surface area) of, for example, 5 m²·g⁻¹ or more, preferably 10 m²·g⁻¹ or more, more preferably 20 m²·g⁻¹ or more, particularly preferably 30 m²·g⁻¹ or more, and for example, 80 m²·g⁻¹ or less, preferably 60 m²·g⁻¹ or less, more preferably 50 m²·g⁻¹ or less. The specific surface area of the methanation reaction catalyst can be measured by the BET method (JIS Z8830:2013).

When the methanation reaction catalyst has a specific surface area of within the above-described range, improvement in catalytic activity can be reliably achieved, and improvement in the methane yield per unit mass can be reliably achieved.

To the methanation reaction catalyst, as necessary, a dilution component, particle component, and binder can be added.

The dilution component is an inert (inactive) substance to the methanation reaction described later, and by adding the dilution component to the methanation reaction catalyst, the temperature control of the methanation reaction catalyst can be made easy.

Examples of the dilution component include alumina (for example, α-alumina, θ-alumina, γ-alumina, etc.), and titania (for example, rutile-form titania, anatase-form titania, etc.), and preferably, γ-alumina is used. The dilution component can be used singly, or can be used in combination of two or more.

The dilution component is added in an amount relative to 100 parts by mass of the methanation reaction catalyst of, for example, 100 parts by mass or more, preferably 1000 parts by mass or more, and for example, 5000 parts by mass or less.

The particle component is, for example, when the methanation reaction catalyst is used in a fluidized bed reactor, a support for supporting the methanation reaction catalyst, and for example, alumina, silica, titania, and monoclinic system zirconia are used. The particle component can be used singly, or can be used in combination of two or more. The amount of the particle component added is selected arbitrarily in accordance with the use of the methanation reaction catalyst.

The binder is a binding component for binding the methanation reaction catalyst together when, for example, the methanation reaction catalyst is used in a fluidized bed reactor, and examples thereof include silicate, titanate, aluminate, and zirconate. The binder can be used singly, or can be used in combination of two or more. The binder can be added in an amount arbitrarily selected in accordance with use of the methanation reaction catalyst.

### (1-2) Method for producing a methanation reaction catalyst

Next, description is given below of an embodiment of the method for producing a methanation reaction catalyst.

The method for producing a methanation reaction catalyst includes a step of mixing material components to prepare a mixture (mixing step), and a step of calcining the mixture to prepare a stabilized zirconia support supporting NiO (calcining step), and as necessary, a step of reducing NiO to Ni (reduction step).

In the mixing step, zirconia (ZrO₂) and/or Zr salt, the above-described salt of the stabilizing element, and Ni salt as the material components are mixed, for example, so that atom percentage of all the atoms (Zr, stabilizing element, and Ni) is in the above-described range.

Examples of the zirconia include low crystalline ZrO₂ fine particles.

Examples of the Zr salt include nitrate of Zr (for example, zirconium nitrate (Zr(NO₃)₄), zirconium nitrate oxide (ZrO(NO₃)₂), etc.), hydrochloride of Zr (for example, zirconium chloride oxide (ZrCl₂O), etc.), and acetate of Zr (for example, zirconium acetate oxide (ZrO(C₂H₃O₂)₂), etc.). The Zr salt can be used singly, or can be used in combination of two or more.

For the Zr salt, a commercially available product can be used, including zirconyl nitrate pentahydrate (manufactured by BOC Sciences), zirconium nitrate oxide dihydrate (manufactured by KANTO CHEMICAL CO.,LTD), zirconium chloride oxide octahydrate (manufactured by KANTO CHEMICAL CO.,LTD), and zirconium acetate oxide (manufactured by DAIICHI KIGENSO KAGAKU KOGYO CO., LTD.).

Of zirconia and Zr salt, preferably, acetate of Zr is used, and even more preferably, zirconium acetate oxide is used.

For the stabilizing element salt, for example, nitrate of the stabilizing element (for example, manganese nitrate (Mn(NO₃)₂), iron nitrate (Fe(NO₃)₃), cobalt nitrate (Co(NO₃)₂), etc.), chloride of the stabilizing element (for example, manganese chloride (MnCl₂), iron chloride (FeCl₃), and cobalt chloride (CoCl₂)) are used. The salt of the stabilizing element can be used singly, or can be used in combination of two or more. For the salt of the stabilizing element, a commercially available product can also be used.

Of the salt of the stabilizing element, preferably, nitrate of the stabilizing element is used, and even more preferably, manganese nitrate, iron nitrate, and cobalt nitrate are used.

Examples of the Ni salt include, for example, nitrate of Ni (for example, nickel nitrate (Ni(NO₃)₂), etc.), and chloride of Ni (for example, nickel chloride (NiCl₂), etc.) are used. Ni salt can be used singly, or can be used in combination of two or more. For the Ni salt, a commercially available product can be used.

Of the Ni salt, preferably, nitrate of Ni is used, and even more preferably, nickel nitrate is used.

To mix the material components, for example, the salt of the stabilizing element and Ni salt are added to zirconia sol and/or an aqueous solution of Zr salt so that the atom percentage of all the atoms (Zr, stabilizing element, and Ni) is within the above-described range, and then the mixture is stirred.

To be more specific, to the zirconia sol and/or aqueous solution of Zr salt, the salt of the stabilizing element is added, and the mixture is stirred to be mixed to prepare a homogenous solution. Thereafter, Ni salt is added, and the mixture is stirred to be mixed for, for example, 1 hour or more and 30 hours or less.

In this manner, a mixture solution containing zirconia and/or Zr salt, salt of the stabilizing element, and Ni salt is prepared.

Then, the mixture solution is heated in, for example, a constant temperature drying oven to evaporate excessive moisture.

The mixture solution is heated at, for example, 100°C or more, preferably 150°C or more, and for example, 200°C or less, preferably 170°C or less. The mixture solution is heated for, for example, 30 minutes or more, preferably 1 hour or more, and for example, 10 hours or less, preferably 3 hours or less.

In this manner, a slurry mixture containing zirconia and/or Zr salt, salt of the stabilizing element, and Ni salt is prepared.

Then, the mixture is stirred as necessary, and then in the calcining step, the mixture is calcined in a heating furnace such as, for example, an electric furnace.

The calcination temperature is 550°C or more, preferably 600°C or more, 800°C or less, preferably 750°C or less, more preferably 700°C or less.

When the calcination temperature is in the above-described lower limit or more, the crystal structure of the stabilized zirconia support can be reliably formed into a tetragonal system and/or cubic system reliably, and when the calcination temperature is the above-described upper limit or less, decrease in catalytic activity by excessive decrease in the specific surface area of the stabilized zirconia support can be suppressed.

In particular, when the stabilizing element is Mn, and the calcination temperature is the above-described lower limit or more, the oxidation number of Mn changes to generate Mn³⁺, and the oxygen vacancy can be reliably formed in the stabilized zirconia support.

The calcination time can be 1 hour or more, preferably 5 hours or more, and for example, 24 hours or less, preferably 10 hours or less.

The mixture is calcined in this manner, and the stabilized zirconia support represented by the above-described general formula (1) is formed, nickel oxide is supported on the stabilized zirconia support, and the methanation reaction catalyst represented by the general formula (6) below is prepared.

### General formula (6):

[Chemical Formula 6]

NiO/Zr⁴⁺_{1-(x+y)}M^{α+}ₓNi²⁺_{y}O_{2-((2-α/2)x+y)} (6)

(in formula (6), x and y are the same range as x and y of general formula(1), M is the transition element of M in general formula (1), α is the same range as that of α of general formula (1).)

That is, the stabilizing element and Ni form a solid solution into the stabilized zirconia support, and nickel oxide is supported on the stabilized zirconia support.

Then, in the reduction step, the methanation reaction catalyst represented by the above-described general formula (6) is reduced by hydrogen flow.

To be more specific, the methanation reaction catalyst represented by the above-described general formula (6) is ground as necessary with a mortar and sieved, and thereafter a predetermined circular tube is loaded with the methanation reaction catalyst.

The sieve has an opening of, for example, 100 µm or less, preferably 75 µm or less.

Then, the circular tube is heated so that the temperature inside thereof is the reduction temperature below, with for example a heater such as an electric tube furnace, and hydrogen is allowed to flow in the circular tube.

Examples of the reduction temperature include 200°C or more, preferably 300°C or more, for example, 600°C or less, preferably 500°C or less. The reduction time is 2 hours or more, preferably 5 hours or more, and for example, 10 hours or less.

The hydrogen flow velocity relative to 1g of the methanation reaction catalyst is, for example, 50 mL·min⁻¹·g⁻¹ or more, preferably 100 mL·min⁻¹·g⁻¹ or more, and for example, 500 mL·min⁻¹·g⁻¹ or less.

In the above-described manner, the nickel oxide supported on the stabilized zirconia support is reduced to a metal state nickel, and the methanation reaction catalyst represented by the general formula (7) below is prepared.

### General formula (7):

[Chemical Formula 7]

Ni/Zr⁴⁺₁-_{(x+y)}M^{α+}ₓNi²⁺_{y}O_{2-((2-α/2)x+y)} (7)

(in formula (7), x and y are the same range as x and y of general formula(1), M is the transition element of M in general formula (1), α is the same range as that of α of general formula (1).)

The stabilizing element and Ni forming a solid solution in the stabilized zirconia support is covered with the metal state nickel supported on the stabilized zirconia support, and therefore is not reduced in the reduction step, and its oxidized state is kept.

The methanation reaction catalyst is in particle state, but it can be formed into a predetermined shape (for example, columnar, prism shape, hollow cylindrical, etc.) by, for example, pressurizing. The methanation reaction catalyst can be supported on the particle component by adding the above-described particle component.

### (1-3) Method for producing methane

Next, description is given below of the method for producing methane in which the above-described methanation reaction catalyst is used.

To produce methane with the methanation reaction catalyst, the methanation reaction catalyst is allowed to contact with a gas mixture containing CO and/or CO₂, and hydrogen gas under the reaction temperature of 200°C or more.

To be more specific, the methanation reaction catalyst is diluted as necessary with the above-described dilution component, and then a predetermined reaction tube is loaded with the methanation reaction catalyst. Then, the temperature of the reaction tube is kept at the reaction temperature below under normal pressure, and a gas mixture is introduced to the reaction tube.

The reaction temperature is 200°C or more, preferably 250°C or more, more preferably 300°C or more, and for example, 500°C or less, preferably 400°C or less.

When the gas mixture contains CO₂ and hydrogen gas, the molar ratio of CO₂ to hydrogen gas is 1:4, and when the gas mixture contains CO and hydrogen gas, the molar ratio of CO to hydrogen gas is 1:3.

The gas mixture flow rate per 1g of the methanation reaction catalyst is, for example, 1000L·h⁻¹·g⁻¹ or more, preferably 2000L·h⁻¹·g⁻¹ or more, and for example, 5000L·h⁻¹·g⁻¹ or less, preferably 4000L·h⁻¹·g⁻¹ or less.

When the methanation reaction catalyst is allowed to contact the gas mixture in this manner, even if the stabilized zirconia support supports NiO, NiO is reduced by hydrogen in the gas mixture, and reduced to a metal state Ni.

Then, the oxygen vacancy of the stabilized zirconia support attracts oxygen atoms of CO and/or CO₂, and the metal state Ni supported on the stabilized zirconia support attracts hydrogen, and therefore on the surface of the methanation reaction catalyst, CO and/or CO₂ are allowed to react with hydrogen efficiently to produce methane.

To be more specific, the methane yield per 1g of the methanation reaction catalyst is, for example, 0.1 mmol·s⁻¹·g⁻¹ or more, preferably 0.5 mmol·s⁻¹·g⁻¹ or more, more preferably 1.8 mmol·s⁻¹·g⁻¹ or more, particularly preferably 2.2 mmol·s⁻¹·g⁻¹ or more, and for example, 10.0 mmol·s⁻¹·g⁻¹ or less, preferably 5.0 mmol·s⁻¹·g⁻¹ or less.

In such a method for producing methane, the metal state Ni supported on the surface of the methanation reaction catalyst may be removed and separated from the methanation reaction catalyst by introduction of the gas mixture. In this case, Ni ion of the stabilized zirconia support exposed at the removed portion is reduced and be in a metal state, and works as a catalytic active site.

### (1-4) Operations and effects

In the methanation reaction catalyst, a specific transition element (Mn, Fe, Co) forms a solid solution into the stabilized zirconia support, and the stabilized zirconia support has a tetragonal system and/or cubic system crystal structure. Such a methanation reaction catalyst can improve the methane yield per unit mass.

Therefore, a desired methane production amount can be secured with a relatively small amount of methanation reaction catalyst. As a result, facility (for example, reaction tube, etc.) size and numbers required for the methanation reaction can be reduced, and facility costs can be reduced.

In the first embodiment, Ni is supported on the stabilized zirconia support, and forms a solid solution into the stabilized zirconia support. Therefore, oxygen vacancies can be reliably formed in the stabilized zirconia support. As a result, improvement in the methane yield per unit mass can be achieved more reliably.

When the stabilizing element is Mn, compared with the case where the stabilizing element is Fe and Co, the methane yield per unit mass can be improved even more reliably.

When the Mn forming the solid solution in the stabilized zirconia support contains at least Mn³⁺, oxygen vacancies can be formed in the stabilized zirconia support more reliably.

When the atomic ratio of the stabilizing element/(Zr + stabilizing element) is within the above-described specific range, improvement in the methane yield per unit mass can be achieved even more reliably.

In the method for producing a methanation reaction catalyst, the above-described methanation reaction catalyst can be produced by mixing zirconia and/or Zr salt, a specific transition element salt, Ni salt, and thereafter calcining the mixture at 550°C or more and 800°C or less.

Therefore, with an easy method, the methanation reaction catalyst that allows for improvement in the methane yield per unit mass can be produced.

In the method for producing methane, the above-described methanation reaction catalyst is allowed to contact with the gas mixture containing CO and/or CO₂ and hydrogen gas, at 200°C or more, and therefore CO and/or CO₂ can be allowed to react with hydrogen gas efficiently, and methane can be produced efficiently.

### 2. Second embodiment

Next, description is given below of the second embodiment of the present invention.

In the first embodiment, Ni is supported on the stabilized zirconia support, and forms solid solution into the stabilized zirconia support, but it is not limited thereto. In the second embodiment, Ni is supported on the stabilized zirconia support, but does not form a solid solution into the stabilized zirconia support.

That is, in the stabilized zirconia support of the second embodiment, only the stabilizing element forms the solid solution, and any of Zr ion and the above-described transition element ion is disposed at a plurality of the lattice points of the stabilized zirconia support. To be more specific, the stabilized zirconia support of the second embodiment is represented by the general formula (8) below.
[Chemical Formula 8]

Zr⁴⁺₁₋ₓM^{α+}ₓO_{2-(2-(2-α/2)x} (8)

(in formula (8), x is the same range as x of general formula(1), M is the transition element of M in general formula (1), α is the same range as that of α of general formula (1).)

That is, the methanation reaction catalyst of the second embodiment includes the stabilized zirconia support including Zr, stabilizing element, and O, and the Ni supported on the stabilized zirconia support.

In the methanation reaction catalyst as well, the atom percentage of the atoms (Zr, stabilizing element, and Ni) relative to a total of atoms is within the above-described range.

Such a methanation reaction catalyst is produced by, for example, mixing zirconia and/or the above-described Zr salt, and the above-described salt of the stabilizing element, and calcining the mixture at the above-described calcining temperature to prepare a calcined product, and then immersing the calcined product in an aqueous solution of the above-described Ni salt, and then again, calcining at the above-described calcination temperature. Thereafter, as necessary, it is reduced in the above-described manner.

The second embodiment can also achieve the same operations and effects of the first embodiment, but in view of the catalytic activity, the first embodiment is preferable.

### 3. Modified example

In the first embodiment and the second embodiment, Ni is supported on the stabilized zirconia support as an active element, but in addition to Ni, Ru can be supported on the stabilized zirconia support. In this manner, further improvement in catalytic activity can be achieved.

Such a methanation reaction catalyst can be prepared by mixing zirconia and/or Zr salt, salt of the stabilizing element, Ni salt, and Ru salt (for example, nitric acid ruthenium(III) solution, etc.) to prepare a mixture, and calcining the mixture at the above-described calcination temperature, or immersing a stabilized zirconia support prepared in advance in an aqueous solution of Ru salt, and then calcining at the above-described calcining temperature.

The above-described first embodiment, second embodiment, and modified example can be suitably combined.

### [Examples]

The present invention is further described in detail based on EXAMPLES below. However, the present invention is not limited to Examples. The specific numerical values of mixing ratio (content), physical property value, and parameter used in the description below can be replaced with the upper limit values (numerical values defined with "or less" or "below") or lower limit values (numerical values defined with "or more" or "more than") of the corresponding numerical values of mixing ratio (content), physical property value, and parameter described in **"DESCRIPTION OF EMBODIMENTS"** above.

### (1) Examples 1 to 3 and Comparative Example 1

An aqueous solution of zirconia acetate salt (trade name: Zircozol ZA-20, manufactured by DAIICHI KIGENSO KAGAKU KOGYO CO., LTD.) is mixed with manganese nitrate hexahydrate (manufactured by KANTO CHEMICAL CO., LTD.) so that the atomic ratio of Mn/(Zr + Mn) was 0.100, and then nickel nitrate hexahydrate (manufactured by KANTO CHEMICAL CO., LTD.) was added so that Ni atom% (Ni/(Zr + Mn + Ni) × 100) relative to a total of Zr, Mn, and Ni was 50 atom%, and the mixture was stirred overnight to prepare a mixture solution.

Then, the mixture solution was put into a constant temperature drying oven having a temperature kept at 170°C and allowed to stand for 2 hours. In this manner, extra moisture was evaporated, thereby preparing a high viscosity slurry mixture (mixture of zirconia acetate salt, manganese nitrate, and nickel nitrate).

Then, the mixture was calcined at the calcination temperature shown in Table 1 for 5 hours, thereby preparing a solid methanation reaction catalyst. Then, the solid methanation reaction catalyst was ground with a mortar, sieved with a sieve with 75 µm under, thereby producing particles of the methanation reaction catalyst.

The methanation reaction catalyst included a stabilized zirconia support into which Ni and Mn formed a solid solution (in the following, referred to as Ni·Mn-solid solution ZrO₂), and NiO supported on the Ni·Mn-solid solution ZrO₂.

Then, glass wool was put in a stainless steel (SUS304) reaction tube (1 inch tube, internal diameter 21 mm), and then the methanation reaction catalyst was introduced therein.

Then, the reaction tube was surrounded by an electric tube furnace, and heated so that the temperature inside the reaction tube was 400°C, and hydrogen was allowed to pass through the reaction tube at a flow velocity of 0.5L/min, and kept for 5 hours. In this manner, NiO supported on the Ni·Mn-solid solution ZrO₂ was reduced to a metal state Ni.

In the above-described manner, a methanation reaction catalyst including the Ni·Mn-solid solution ZrO₂ and metal state Ni supported on the Ni·Mn-solid solution ZrO₂ (Ni/Ni·Mn-solid solution ZrO₂) was prepared.

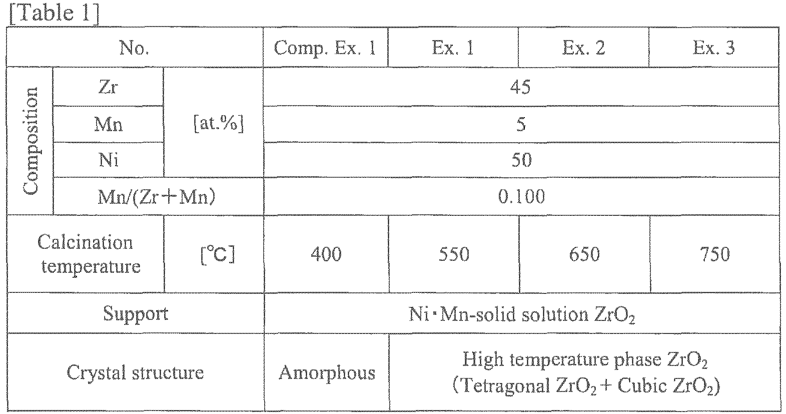

### <Measurement and evaluation>

### (1-1) Methane yield

10 mg of the methanation reaction catalyst of Examples 1 to 3 and Comparative Example 1 was mixed and diluted in advance with 4.5g of γ alumina, and the mixture was loaded into a reaction tube (SUS304 tube, internal diameter 15 mm × height 100 mm).

Then, the temperature of reaction tube was kept at 250°C, 300°C, 350°C, or 400°C (reaction temperature) under normal pressure, and a material gas (gas mixture) including carbon dioxide, hydrogen, and nitrogen was fed to the reaction tube, thereby allowing contact with the methanation reaction catalyst.

In the material gas, hydrogen/carbon dioxide = 4(molar ratio), and nitrogen was 5% by volume. The flow rate of the material gas was 0.5L/min, which was 3000L·h⁻¹·g⁻¹ per 1g of the catalyst.

Then, after the contact with the methanation reaction catalyst, the reaction gas flowing out from the reaction tube was analyzed with a thermal conductivity detector (TCD) gas chromatography. The reaction gas contained only unreacted hydrogen, unreacted carbon dioxide, and a product methane.

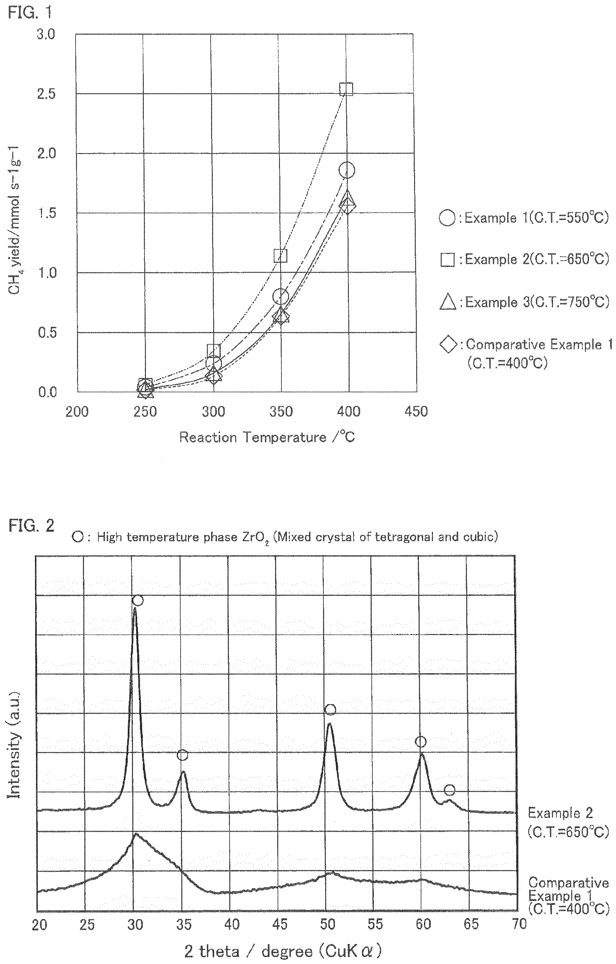

Based on the methane content of the reaction gas, methane yield per 1g of the catalyst (CH₄ yield unit: mmol·s⁻¹·g⁻¹) was calculated. The results are shown in FIG. 1. In FIG. 1, the horizontal axis shows reaction temperature (unit:°C), and the vertical axis shows methane yield per unit mass (unit: mmol·s⁻¹·g⁻¹). In FIG. 1, the calcination temperature is shown as C.T.

FIG. 1 shows that the methanation reaction catalyst (Examples 1 to 3) with the calcination temperature of 550°C or more had a higher catalytic activity than that of the methanation reaction catalyst (Comparative Example 1) with the calcination temperature of 500°C or less. In particular, it shows that when the calcination temperature was around 650°C, activity of the methanation reaction catalyst significantly improved.

### (1-2) X-ray diffraction (XRD)

The methanation reaction catalyst of Example 2 and Comparative Example 1 was analyzed by X-ray diffraction (glancing angle 10°, Cu-Kα). The results are shown in FIG. 2. In FIG. 2, the peak corresponding to the high temperature phase ZrO₂ (mixed crystal of ZrO₂ having a tetragonal system crystal structure and ZrO₂ having a cubic system crystal structure) is shown with ○.

FIG. 2 shows that the methanation reaction catalyst with the calcination temperature of 650°C (Example 2) contained high temperature phase ZrO₂. Meanwhile, the methanation reaction catalyst having a calcination temperature of 400°C (Comparative Example 1) did not contain the high temperature phase ZrO₂ clearly, and contained a low crystalline ZrO₂, or oxide with a not completely formed solid solution (manganese oxide and nickel oxide).

That is, FIG. 1 and FIG. 2 show that as the calcination temperature increases, crystallinity of ZrO₂ improves, and by the methanation reaction catalyst containing the high temperature phase ZrO₂, catalytic activity can be reliably improved.

### (1-3) BET specific surface area

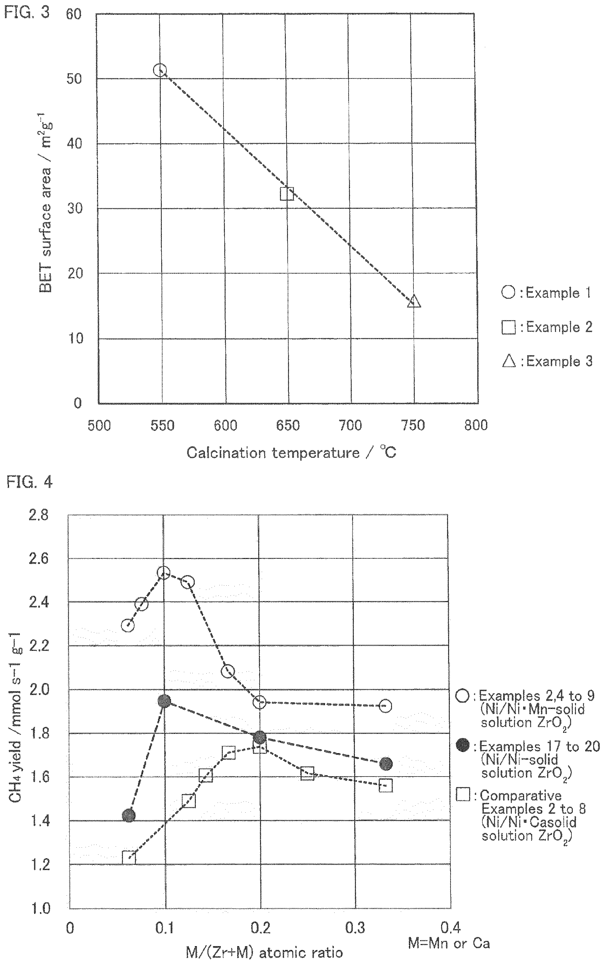

The specific surface area of the methanation reaction catalyst of Examples 1 to 3 was measured in accordance with BET method. The results are shown in FIG. 3. In FIG. 3, the horizontal axis shows calcination temperature (unit:°C), and the vertical axis shows BET specific surface area (unit:m²·g⁻¹).

FIG. 3 shows that as the calcination temperature increases, grain growth is caused, and the specific surface area of the methanation reaction catalyst decreases.

That is, FIG. 1 and FIG. 3 show that when the calcination temperature is 750°C or less, particularly 650°C or less, excessive decrease in the specific surface area can be suppressed, and improvement in catalytic activity can be reliably achieved.

### (2) Examples 4 to 20 and Comparative Examples 2 to 21

### •Examples 4 to 9

A methanation reaction catalyst was prepared in the same manner as in Example 2 (calcination temperature 650°C), except that an aqueous solution of zirconia acetate salt was mixed with manganese nitrate hexahydrate so that the atomic ratio of Mn/(Zr + Mn) was as shown in Table 2.

### •Examples 10 to 16

A methanation reaction catalyst was prepared in the same manner as in Examples 2, 4 to 9 except that nickel nitrate hexahydrate was added so that the Ni atom% (Ni/(Zr + Mn + Ni) × 100) was 70 atom%.

### •Examples 17 to 20

An aqueous solution of zirconia acetate salt was mixed with manganese nitrate hexahydrate so that the atomic ratio of Mn/(Zr + Mn) was as shown in Table 2, and then the mixture was put in a constant temperature drying oven kept at 170°C, and allowed to stand for 2 hours. In this manner, a mixture of zirconia acetate salt and manganese nitrate was prepared.

Then, the mixture was calcined at 650°C for 5 hours, and a stabilized zirconia support into which only Mn formed a solid solution (in the following, referred to as Mn-solid solution ZrO₂) was prepared. Then, the Mn-solid solution ZrO₂ was ground with a mortar.

Then, the ground Mn-solid solution ZrO₂ was added to an aqueous solution of nickel nitrate prepared so that the Ni atom% (Ni/(Zr + Mn + Ni) × 100) was 50 atom%, and after subjecting it to a vacuum environment for 1 hour, it was allowed to stand for 72 hours for immersion. In this manner, a Mn-solid solution ZrO₂ supporting nickel nitrate was prepared.

Then, the Mn-solid solution ZrO₂ was calcined at 650°C (calcination temperature) for 5 hours, thereby producing a methanation reaction catalyst. The methanation reaction catalyst included the Mn-solid solution ZrO₂, and NiO supported on the Mn-solid solution ZrO₂.

Then, in the same manner as in Example 1, the NiO supported on the Mn-solid solution ZrO₂ was reduced to a metal state Ni.

In the above-described manner, a methanation reaction catalyst including the Mn-solid solution ZrO₂ and a metal state Ni supported on the Mn-solid solution ZrO₂ (Ni/Mn-solid solution ZrO₂) was prepared.

### •Comparative Examples 2 to 8

A methanation reaction catalyst was prepared in the same manner as in Examples 2, 4 to 9, except that manganese nitrate hexahydrate was changed to calcium nitrate tetrahydrate, and an aqueous solution of zirconia acetate salt was mixed with calcium nitrate tetrahydrate so that the atomic ratio of Ca/(Zr + Ca) was as shown in Table 3. The methanation reaction catalyst included the stabilized zirconia support (in the following, referred to as Ni·Ca-solid solution ZrO₂) into which Ni and Ca formed a solid solution, and a metal state Ni supported on Ni·Ca-solid solution ZrO₂ (Ni/Ni·Ca-solid solution ZrO₂).

### •Comparative Examples 9 to 14

A methanation reaction catalyst was prepared in the same manner as in Comparative Examples 3 to 8, except that nickel nitrate hexahydrate was added so that the Ni atom%(Ni/(Zr + Mn + Ni) × 100) was 70 atom%. The methanation reaction catalyst included the Ni·Ca-solid solution ZrO₂, and a metal state Ni supported on the Ni·Ca-solid solution ZrO₂.

### •Comparative Examples 15 to 21

The Mn-solid solution ZrO was prepared in the same manner as in Examples 17 to 20, and ground with a mortar. The Mn-solid solution ZrO was used as the methanation reaction catalyst as is without immersing it in the aqueous solution of nickel nitrate. The methanation reaction catalyst included no Ni, and consisted of the Mn-solid solution ZrO₂ (Ni free Mn-solid solution ZrO₂).

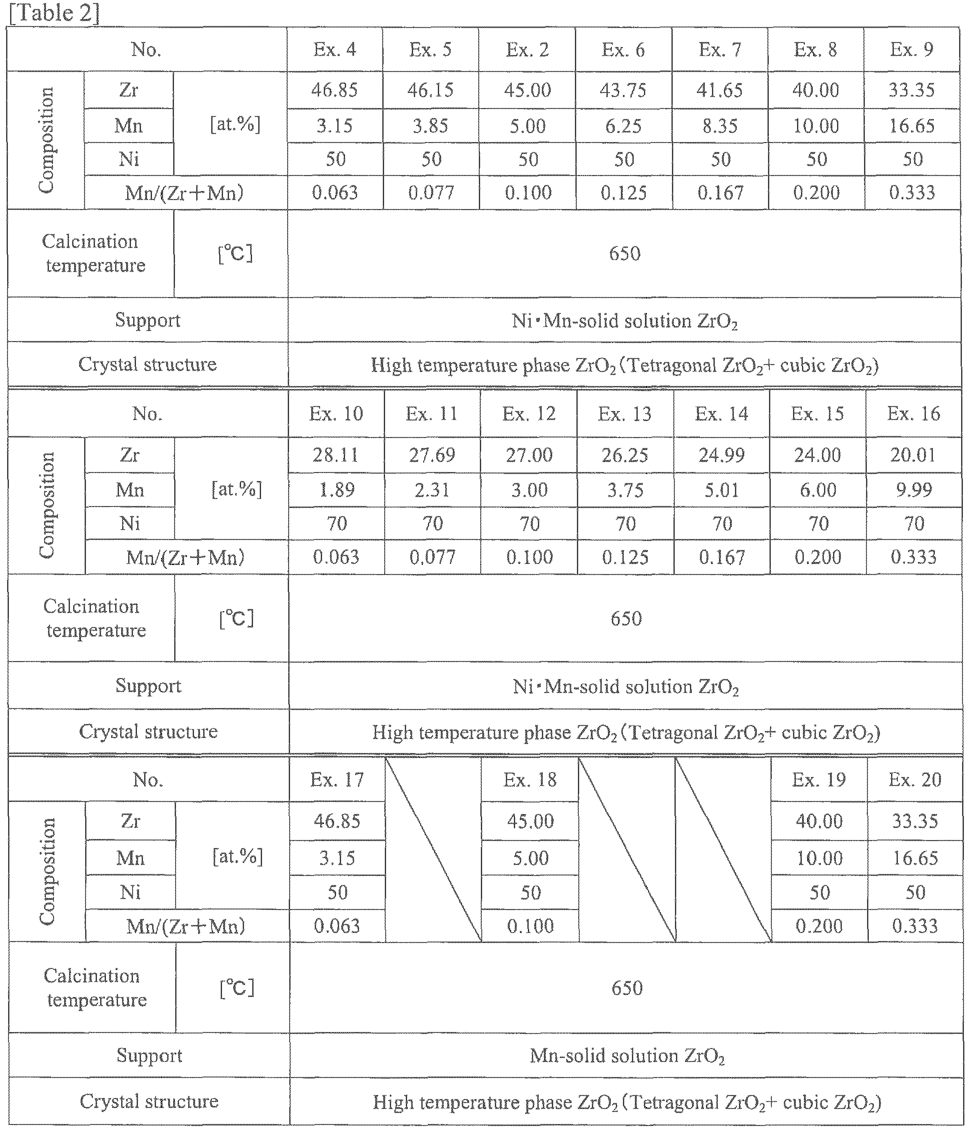

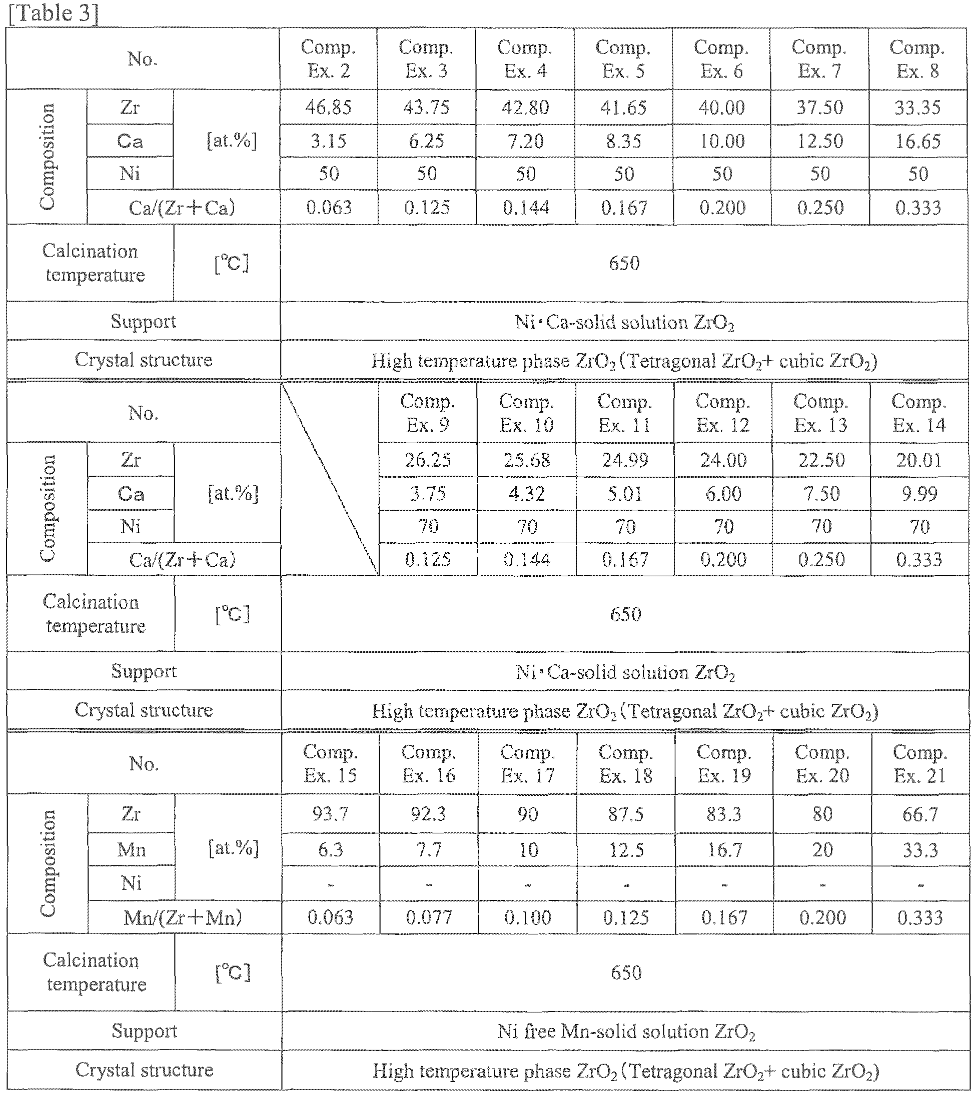

### <Measurement and evaluation>

### (2-1) Methane yield

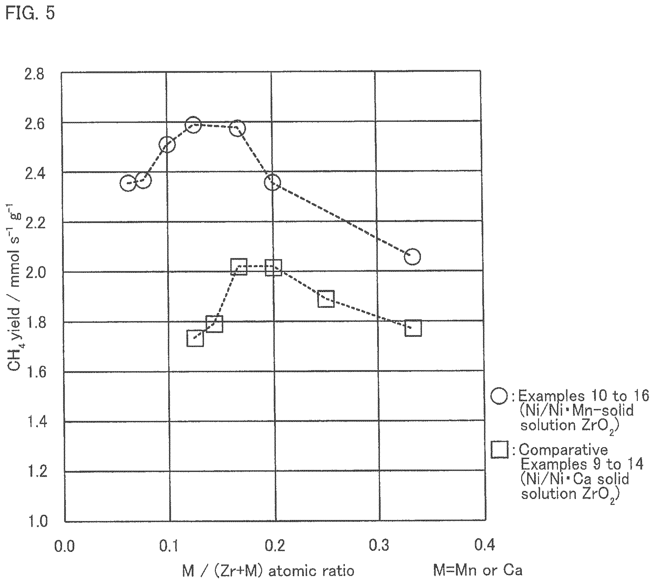

The methane yield of the methanation reaction catalyst of Examples 2, 4 to 20 and Comparative Examples 2 to 14 was measured in the same manner as in the above-described measurement of the methane yield at the reaction temperature of 400°C. The results for Examples 2, 4 to 9, 17 to 20 and Comparative Examples 2 to 8 are shown in FIG. 4, and the results for Examples 10 to 16 and Comparative Examples 9 to 14 are shown in shown in FIG. 5. In FIG. 4 and FIG. 5, the horizontal axis shows the atomic ratio of M/(Zr + M), and the vertical axis shows methane yield per unit mass (unit:mmol·s⁻¹g⁻¹).

FIG. 4 showed that with the methanation reaction catalyst containing the Ni/Ni·Mn-solid solution ZrO₂ (Examples 2, 4 to 9) and the methanation reaction catalyst containing the Ni/Mn-solid solution ZrO₂ (Examples 17 to 20), in the Mn/(Zr + Mn) range of 0.063 or more and 0.333 or less, catalytic activity was high compared with the methanation reaction catalyst containing Ni/Ni·Ca-solid solution ZrO₂ (Comparative Examples 2 to 8).

The methanation reaction catalyst containing Ni/Ni Mn-solid solution ZrO₂ (Examples 2, 4 to 9) had a higher catalytic activity than that of the methanation reaction catalyst containing Ni/Mn-solid solution ZrO₂ (Examples 17 to 20).

FIG. 5 showed that even if the Ni atom% was 70 atom%, the methanation reaction catalyst containing the Ni/Ni·Mn-solid solution ZrO₂ (Examples 10 to 16) had higher catalytic activity than that of methanation reaction catalyst containing Ni/Ni·Ca-solid solution ZrO₂ (Comparative Examples 9 to 14).

### (2-2) XRD

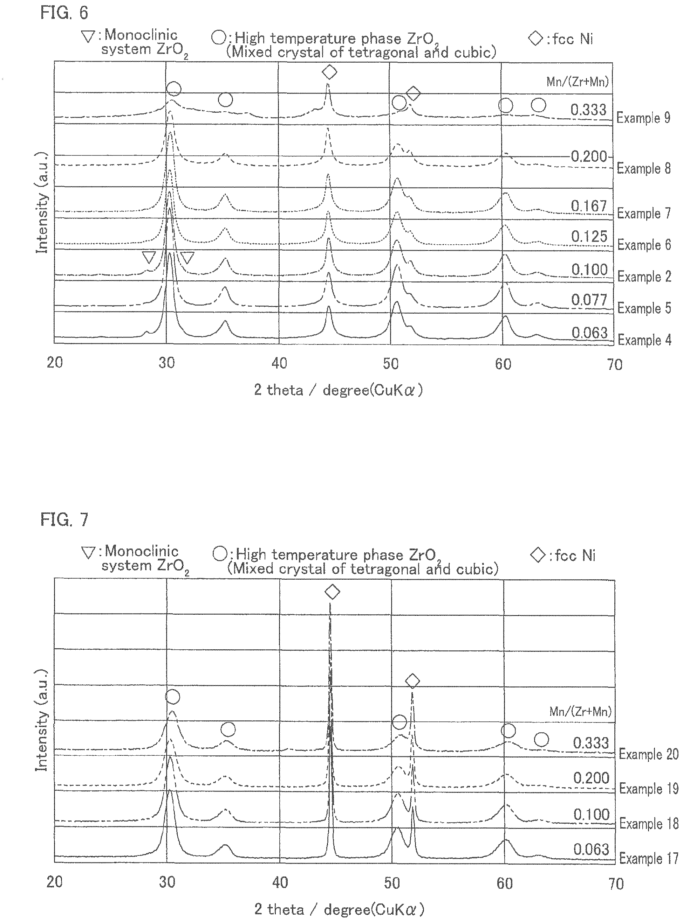

The methanation reaction catalyst of Examples 2, 4 to 9 and 17 to 20 was analyzed with X-ray diffraction (glancing angle 10°, Cu-Kα). FIG. 6 shows the X-ray diffraction pattern of the methanation reaction catalyst of Examples 2, 4 to 9, and FIG. 7 shows the X-ray diffraction pattern of the methanation reaction catalyst of Examples 17 to 20. In FIG. 6 and FIG. 7, the peak corresponding to the monoclinic system ZrO₂ is shown with ∇, the peak corresponding to high temperature phase ZrO₂ is shown with ○, and the peak corresponding to fcc Ni is shown with ◇.

As shown in FIGs. 6 and 7, with the methanation reaction catalyst containing the Ni/Ni·Mn-solid solution ZrO₂ (ref: Examples 2, 4 to 9, and FIG. 6), compared with the methanation reaction catalyst containing the Ni/Mn-solid solution ZrO₂ (ref: Examples 17 to 20, FIG. 7), the diffraction ray of the high temperature phase ZrO₂ (111 plane) 2θ ≒ 30.3° slightly shifted to a wider angle side.

The methanation reaction catalyst containing the Ni/Ni·Mn-solid solution ZrO₂ (ref: Examples 2, 4 to 9, FIG. 6) had a non-steep diffraction ray and had a smaller particle size compared with the methanation reaction catalyst containing Ni/Mn-solid solution ZrO₂ (Examples 17 to 20).

### (2-3) Crystal lattice spacing of [111] planes of stabilized ZrO₂ support

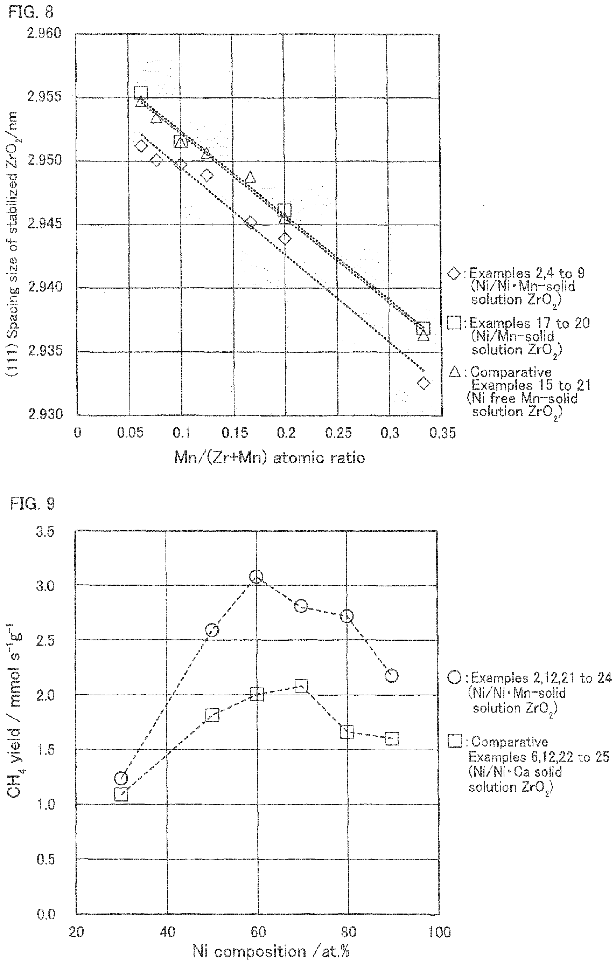

For the methanation reaction catalyst of Examples 2, 4 to 9, 17 to 20 and Comparative Examples 15 to 21, the crystal lattice spacing of [111] planes of the stabilized ZrO₂ support was calculated using Bragg's formula based on the angle for 111 diffraction ray obtained by powder X-ray diffraction method. The results are shown in FIG. 8. In FIG. 8, the horizontal axis shows the atomic ratio of Mn/(Zr + Mn), and the vertical axis shows the crystal lattice spacing of [111] planes of the stabilized ZrO₂ support (unit: nm).

FIG. 8 shows that the methanation reaction catalyst containing the Ni/Ni·Mn-solid solution ZrO₂ (Examples 2, 4 to 9) had a smaller crystal lattice spacing of [111] planes of the stabilized ZrO₂ support than that of the Ni free Mn-solid solution ZrO₂ (Comparative Examples 15 to 21). That is, in the Ni·Mn-solid solution ZrO₂, Ni having a smaller ionic radius than that of Zr formed a solid solution into ZrO₂.

Meanwhile, the methanation reaction catalyst containing the Ni/Mn-solid solution ZrO₂ (Examples 17 to 20) had almost the same crystal lattice spacing of [111] planes of the stabilized ZrO₂ support, compared with the Ni free Mn-solid solution ZrO₂ (Comparative Examples 15 to 21). That is, in the Ni/Mn-solid solution ZrO₂, Ni did not form a solid solution into the ZrO₂. The Ni/Ni·Mn-solid solution ZrO₂ clearly had a different structure from that of the Ni/Mn-solid solution ZrO₂.

### (3) Examples 21 to 24 and Comparative Examples 22 to 25

### •Examples 21 to 24

A methanation reaction catalyst was prepared in the same manner as in Example 2 (Mn/(Zr + Mn)=0.100) except that nickel nitrate hexahydrate was added so that the Ni atom% (Ni/(Zr + Mn + Ni) × 100) was as shown in Table 4.

### •Comparative Examples 22 to 25

A methanation reaction catalyst was prepared in the same manner as in Comparative Example 6 (Ca/(Zr + Ca)=0.200), except that nickel nitrate hexahydrate was added so that the Ni atom% (Ni/(Zr + Ca + Ni) × 100) was as shown in Table 4.

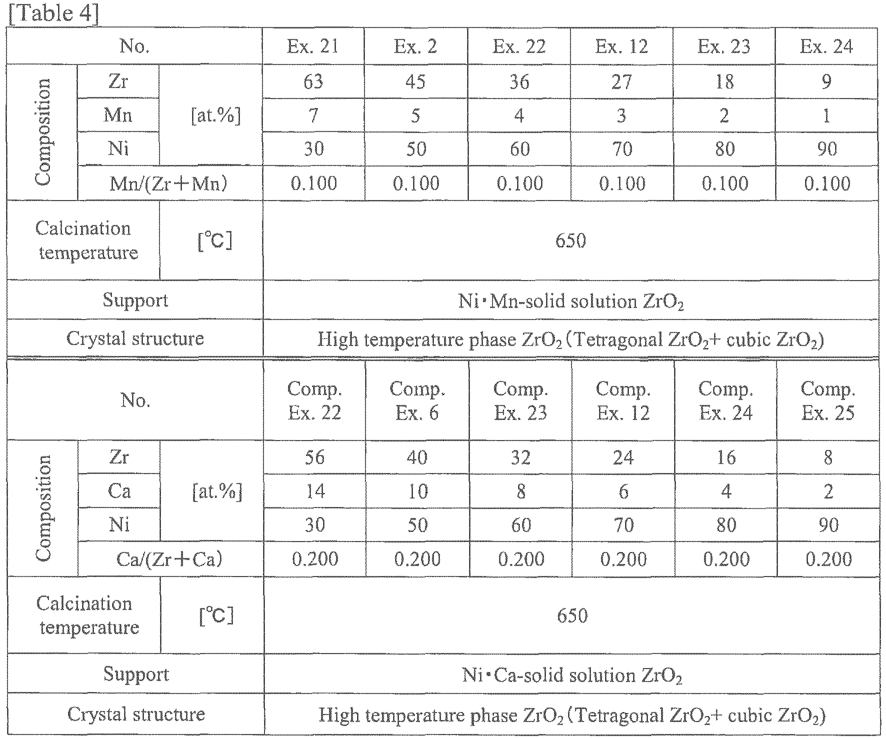

### <Measurement and evaluation>

### (3-1) Methane yield

The methane yield of the methanation reaction catalyst of Examples 2, 12, 21 to 24 and Comparative Examples 6, 12, 22 to 25 was measured in the same manner as in the above-described measurement of the methane yield at a reaction temperature of 400°C. The results are shown in FIG. 9. In FIG. 9, the horizontal axis shows Ni atom% (at.%), and the vertical axis shows methane yield per unit mass (unit: mmol·s⁻¹·g⁻¹).

In FIG. 9, the methanation reaction catalyst containing Ni/Ni·Mn-solid solution ZrO₂ (Examples 2, 12, 21 to 21) had a higher catalytic activity in the range of the Ni atom% of 30 atom% or more and 90 atom% or less than that of the methanation reaction catalyst containing the Ni/Ni·Ca-solid solution ZrO₂ (Comparative Examples 6, 12, 22 to 25).

In particular, the methanation reaction catalyst containing the Ni/Ni·Mn-solid solution ZrO₂ can secure the methane yield of 2.5 mmol·s⁻¹·g⁻¹ or more in the range of the Ni atom% of 50 atom% or more and 80 atom% or less.

### (4) Examples 25 to 32 and Comparative Example 26

### •Examples 25 to 28

A methanation reaction catalyst was prepared in the same manner as in Examples 2, 4, 8, and 9, except that manganese nitrate hexahydrate was changed to cobalt nitrate hexahydrate, and an aqueous solution of zirconia acetate salt was mixed with cobalt nitrate hexahydrate so that the atomic ratio of Co/(Zr + Co) was as shown in Table 5. The methanation reaction catalyst included the stabilized zirconia support (in the following, referred to as Ni·Co-solid solution ZrO₂) into which Ni and Co formed a solid solution, and a metal state Ni supported on Ni·Co-solid solution ZrO₂ (Ni/Ni·Co-solid solution ZrO₂).

### •Examples 29 to 32

A methanation reaction catalyst was prepared in the same manner as in Examples 2, 4, 8, and 9, except that manganese nitrate hexahydrate was changed to iron nitrate nonahydrate, and an aqueous solution of zirconia acetate salt was mixed with iron nitrate nonahydrate so that the atomic ratio of Fe/(Zr + Fe) was as shown in Table 5. The methanation reaction catalyst included the stabilized zirconia support (in the following, referred to as Ni·Fe-solid solution ZrO₂) into which Ni and Fe formed a solid solution, and a metal Ni supported on the Ni·Fe-solid solution ZrO₂ (Ni/Ni·Fe-solid solution ZrO₂).

### •Comparative Example 26

Nickel oxide was reduced in the same manner as described above to a metal state Ni, and used as the methanation reaction catalyst.

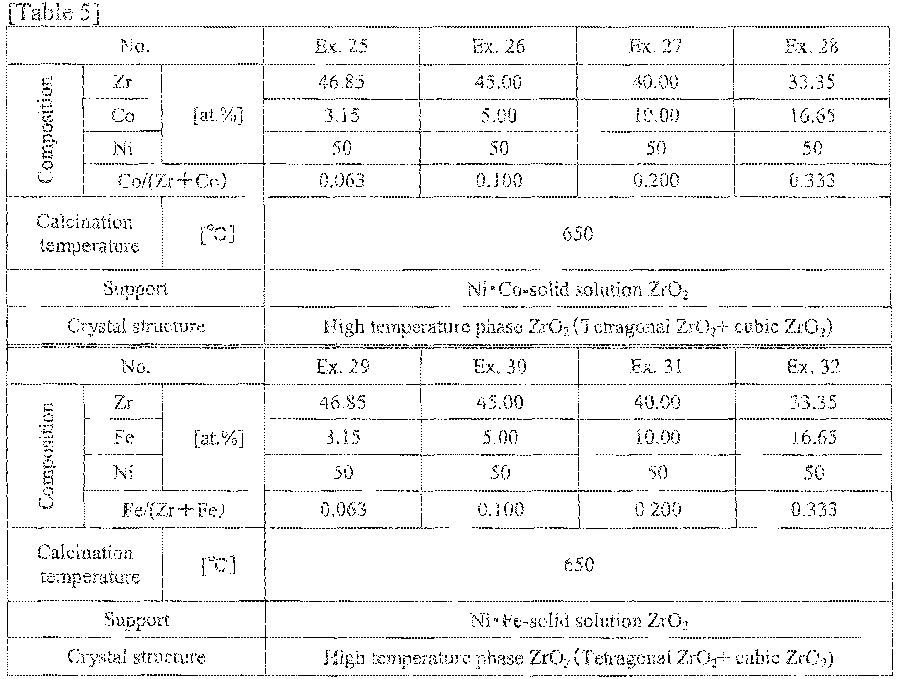

### <Measurement and evaluation>

### (4-1) Methane yield

### •CO₂ methanation

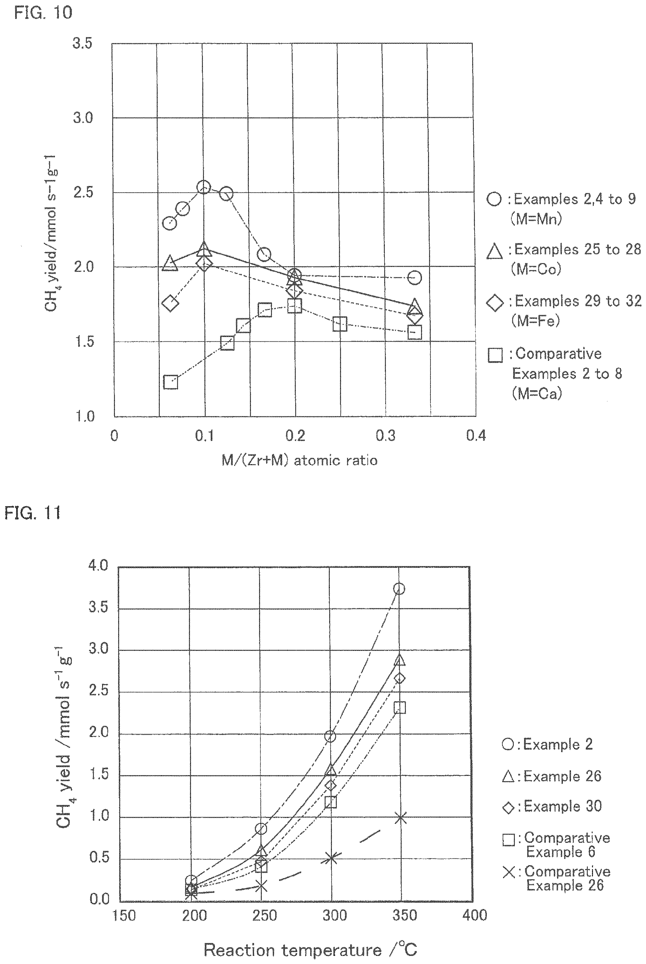

The methane yield of the methanation reaction catalyst of Examples 25 to 32 was measured in the same manner as in the above-described measurement of the methane yield at a reaction temperature of 400°C. The results are shown in FIG. 10. In FIG. 10, the horizontal axis shows the atomic ratio of M/(Zr + M), and the vertical axis shows methane yield per unit mass (unit: mmol·s⁻¹·g⁻¹). FIG. 10 shows, for comparison, the methane yield of Examples 2, 4 to 9 and Comparative Examples 2 to 8.

In FIG. 10, the methanation reaction catalyst containing Ni/Ni·Co-solid solution ZrO₂ (Examples 25 to 28) and the methanation reaction catalyst containing the Ni/Ni·Fe-solid solution ZrO₂ (Examples 29 to 32) had a higher catalytic activity in the range of the atomic ratio of M/(Zr + M) of 0.063 or more and 0.333 or less, compared with the methanation reaction catalyst containing the Ni/Ni·Ca-solid solution ZrO₂ (Comparative Examples 2 to 8).

Comparison of the catalytic activity between the stabilizing element showed the results of Mn (Examples 2, 4 to 9) > Co (Examples 25 to 28) > Fe (Examples 29 to 32).

### •CO methanation

The methane yield of the methanation reaction catalyst of Examples 2, 26, and 30, and Comparative Examples 6 and 26 was measured in the same manner as in the above-described measurement of the methane yield, except that the material gas containing carbon dioxide was changed to the material gas below.

Material gas: contained hydrogen, carbon monoxide, and nitrogen. Hydrogen/carbon monoxide = 3 (molar ratio), nitrogen 5% by volume.

The results are shown in FIG. 11. In FIG. 11, the horizontal axis shows reaction temperature (unit:°C), and the vertical axis shows methane yield per unit mass (unit: mmol·s⁻¹·g⁻¹).

FIG. 11 shows that with the methanation reaction catalyst of Examples and Comparative Examples (Examples 2, 26, and 30, and Comparative Examples 6 and 26), the CO methanation also showed the same tendency with that of CO₂ methanation. To be specific, comparison of the catalytic activity between the stabilizing element showed the results of Mn (Example 2) > Co (Example 26) > Fe (Example 30) > Ca (Comparative Example 6).

The CO methanation showed higher reaction velocity than that of the CO₂ methanation, and reaction progressed even under low temperature. With the methanation reaction catalyst consisting of a metal state Ni (Comparative Example 26), CO methanation also progresses, but the methanation reaction catalyst including the stabilized ZrO₂ support had an even higher catalytic activity.

### (4-2) XRD

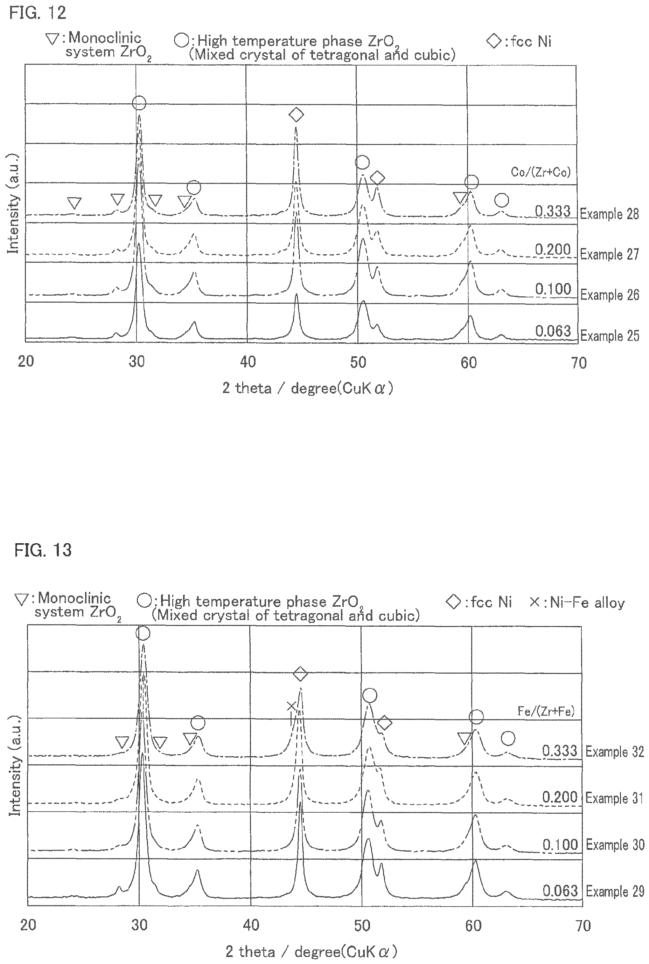

The methanation reaction catalyst of Examples 25 to 32 was analyzed by X-ray diffraction (glancing angle 10°, Cu-Kα). FIG. 12 shows the X-ray diffraction pattern of the methanation reaction catalyst of Examples 25 to 28 (Ni/Ni·Co-solid solution ZrO₂), and FIG. 13 shows the X-ray diffraction pattern of the methanation reaction catalyst of Examples 29 to 32 (Ni/Ni·Fe-solid solution ZrO₂).

In FIG. 12 and FIG. 13, the peak corresponding to the monoclinic system ZrO₂ is shown with ∇, the peak corresponding to the high temperature phase ZrO₂ is shown with ○, the peak corresponding to the fcc Ni is shown with ◇, and the peak corresponding to the Ni-Fe alloy is shown with X.

As shown in FIGs. 12 and 13, even if the stabilizing element is Co (ref: Examples 25 to 28, FIG. 12) or Fe (ref: Examples 29 to 32, FIG. 13), the stabilizing element formed a solid solution into ZrO₂, and the high temperature phase ZrO₂ was formed.

Meanwhile, when the stabilizing element is Co (ref: FIG. 12) or Fe (ref: FIG. 13), the monoclinic system ZrO₂ was contained compared with the case where the stabilizing element was Mn (ref: FIG. 6).

When the stabilizing element is Fe, and Fe/(Zr + Fe) was 0.333 (ref: FIG. 13), in addition to the monoclinic system ZrO₂, the peak corresponding to Ni-Fe alloy (2θ ≒ 43.7°) was observed. The Ni-Fe alloy was produced probably at the stage of hydrogen reduction. This shows that the catalytic activity has the above-described tendency (Mn>Co>Fe).

### (4-3) Crystal lattice spacing of [111] planes of stabilized ZrO₂ support

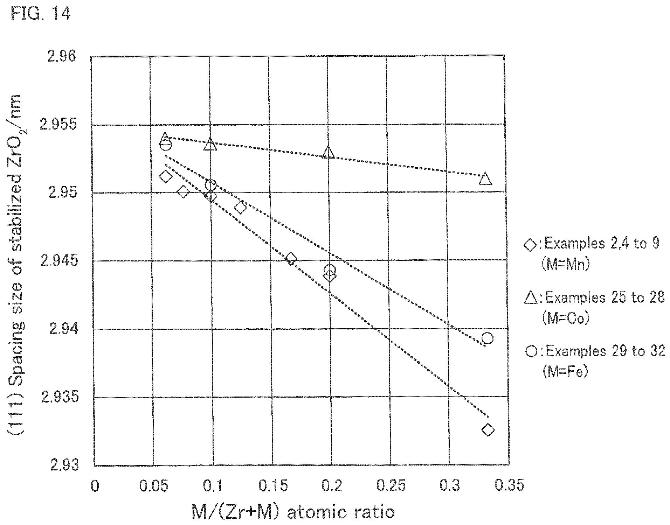

For the methanation reaction catalyst of Examples 2, 4 to 9, 25 to 32, crystal lattice spacing of [111] planes of the stabilized ZrO₂ support was calculated as in the above-described manner. The results are shown in FIG. 14. In FIG. 14, the horizontal axis shows the atomic ratio of M/(Zr + M), and the vertical axis shows the crystal lattice spacing of [111] planes of the stabilized ZrO₂ support (unit:nm).

In FIG. 14, with the stabilizing element of any of Mn (Examples 2, 4 to 9), Co (Examples 25 to 28), and Fe (Examples 29 to 32), as the atomic ratio of M/(Zr + M) increases, the crystal lattice spacing of [111] planes of the stabilized ZrO₂ support decreased. This showed that the stabilizing element of any of Mn, Co, and Fe formed a solid solution into ZrO₂ with the stabilizing element (Mn, Co, Fe).

Furthermore, with the transition of the crystal lattice spacing of the stabilized ZrO₂ support, relationship with the ionic radius of the stabilizing element is assumed to be Mn ≒ Fe<Co.

To be more specific, the Ni·Fe-solid solution ZrO₂ (Examples 29 to 32) was red-brown powder, and has a basic structure of Fe₂O₃ + ZrO₂. That is, Fe is assumed to be Fe³⁺. Fe³⁺ has an ionic radius of 0.0645 nm.

The Ni·Co-solid solution ZrO₂ (Examples 25 to 28) was a grey powder, and because it does not show deliquescence, CoO or Co₃O₄ + ZrO₂ is the basic structure. Co has an ionic radius larger than the ionic radius of Fe, and is closer to the ionic radius of Zr⁴⁺ (0.079 nm), and therefore Co was Co²⁺ having an ionic radius of 0.0745 nm.

### (5) X-ray photoelectron spectroscopy (XPS)

### (5-1) Mn 2p orbital

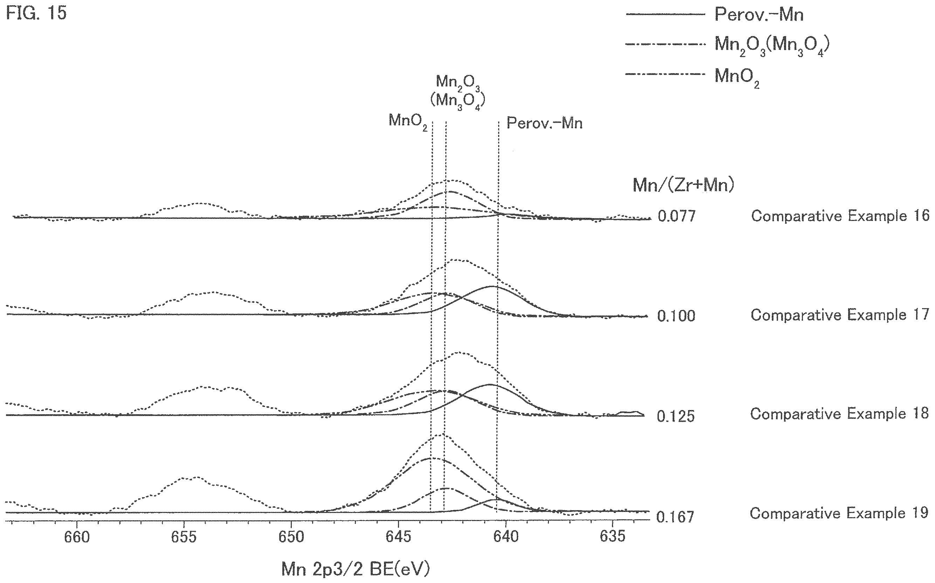

The methanation reaction catalyst of Comparative Examples 16 to 19 (Ni free Mn-solid solution ZrO₂) was analyzed by X-ray photoelectron spectroscopy (XPS). The spectrum corresponding to Mn 2p orbital is shown in FIG. 15.

In FIG. 15, the peak corresponding to a perovskite composite oxide (composite oxide in which oxygen in oxide is shared by two elements) (Perov.-Mn) was observed. The peak of the perovskite composite oxide is assumed to increase as Mn forms a solid solution into ZrO₂. In particular, when Mn/(Zr + Mn) is 0.100 or more and 0.125 or less, the peak of the perovskite composite oxide increased.

When Mn/(Zr + Mn) is more than 0.125 (for example, 0.167), the peak of the perovskite composite oxide decreased, and the peak corresponding to MnO₂ and Mn₂O₃(Mn₃O₄) increased. That is, excessive addition of Mn may possibly suppress the solid solution formation of Mn into ZrO₂.

### (5-2) Mn 3s orbital

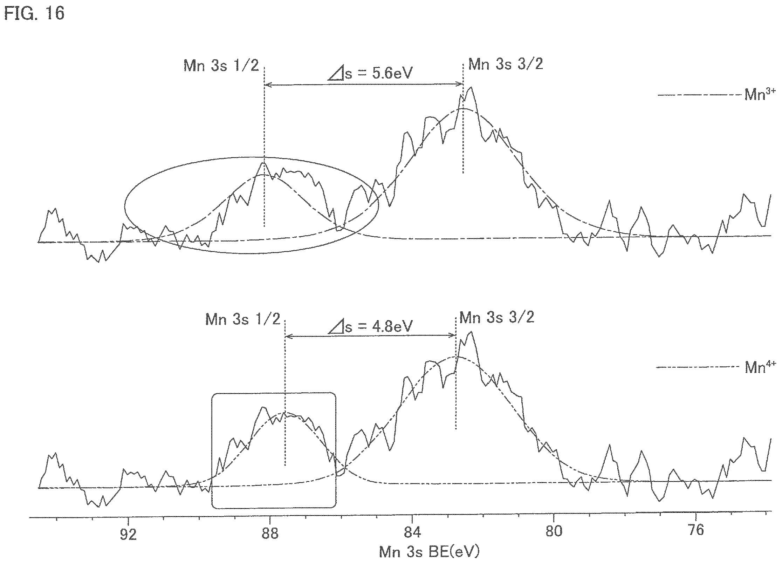

The spectrum corresponding to Mn 3s orbital of the methanation reaction catalyst of Comparative Example 18 (Ni free Mn-solid solution ZrO₂) is shown in FIG. 16.

In FIG. 16, based on comparison between the actually measured peak and theoretical peak of Mn³⁺ (upper side), and comparison between the actually measured peak and the theoretical peak of Mn⁴⁺ (lower side), Mn valence contained in the Mn-solid solution ZrO₂ was assumed. In the actually measured peak, Mn³⁺ peak overlaps with Mn⁴⁺ peak, and Mn contained in the Mn-solid solution ZrO₂ was assumed to contain both Mn³⁺ and Mn⁴⁺.

While the illustrative embodiments of the present invention are provided in the above description, such is for illustrative purpose only and it is not to be construed as limiting in any manner. Modification and variation of the present invention that will be obvious to those skilled in the art is to be covered by the following claims.

### [Industrial Applicability]

The methanation reaction catalyst and the method for producing methane of the present invention are suitably used for a methanation device of CO and/or CO₂. The method for producing a methanation reaction catalyst of the present invention is suitably used for production of a methanation reaction catalyst.

## Claims

1. A methanation reaction catalyst for methanation by allowing CO and/or CO₂ to react with hydrogen,
wherein the methanation reaction catalyst includes
a stabilized zirconia support, into which a stabilizing element forms a solid solution, and having a crystal structure of a tetragonal system and/or cubic system, and
Ni supported on the stabilized zirconia support, and
the stabilizing element is a transition element of at least one selected from the group consisting of Mn, Fe, and Co.

2. The methanation reaction catalyst according to Claim 1,
wherein relative to a total of Zr composing the stabilized zirconia support, the stabilizing element, and Ni,
the atom percentage of Zr is 6.5 atom% or more and 66.5 atom% or less,
the atom percentage of the stabilizing element is 0.50 atom% or more and 24.5 atom% or less, and
the atom percentage of Ni is 30.0 atom% or more and 90.0 atom% or less.

3. The methanation reaction catalyst according to Claim 1,
wherein the Ni is supported on the stabilized zirconia support, and also forms a solid solution into the stabilized zirconia support.

4. The methanation reaction catalyst according to Claim 1, wherein the stabilizing element is Mn.

5. The methanation reaction catalyst according to Claim 4, wherein Mn includes at least Mn³⁺.

6. The methanation reaction catalyst according to Claim 1, wherein the atomic ratio of the stabilizing element/(Zr + the stabilizing element) is 0.05 or more and 0.35 or less.

7. A method for producing methane, the method including:
allowing the methanation reaction catalyst according to Claim 1 to contact a gas mixture containing CO and/or CO₂ and hydrogen gas at 200°C or more.

8. A method for producing a methanation reaction catalyst, the method including the steps of:
preparing a mixture by mixing zirconia and/or Zr salt, a salt of the stabilizing element, and Ni salt, and
calcining the mixture at 550°C or more and 800°C or less,
wherein the stabilizing element is a transition element of at least one selected from the group consisting of Mn, Fe, and Co.
